# NOUVEAU FASCICULE DE BREVET EUROPEEN

(11) **EP 0 820 767 B2**
(45) Date de publication et mention de la décision concernant l'opposition: **25.10.2006**
(45) Mention de la délivrance du brevet: 09.10.2002
(21) Numéro de dépôt: 97401650.3
(22) Date de dépôt: 09.07.1997
(51) Int. Cl.: A61Q 19/00

(54) **Utilisation de dérivés de la mélatonine pour la dépigmentation de la peau et compositions les comprenant**
Verwendung von Melatonin-Derivaten zur Hautdepigmentierung sowie diese enthaltende Präparate
Use of melatonin derivatives for skin depigmentation and compositions comprising the same

(30) Priorité: 25.07.1996 FR 9609388
(43) Date de publication de la demande: 28.01.1998
(73) Titulaire: L'ORÉAL, 75008 Paris (FR)
(72) Inventeur: Nguyen, Quang Lan, 92160 Antony (FR); Nadaud, Jean-François, 92140 Clamart (FR)
(74) Mandataire: Kromer, Christophe

(56) Documents cités:
- EP-A2- 0 438 856
- EP-B1- 0 214 254
- WO-A-92/01810
- JP-A- 61 221 104
- PATENT ABSTRACTS OF JAPAN vol. 011, no. 060 (C-405), 24 février 1987 & JP 61 221104 A (SHISEIDO CO LTD), 1 octobre 1986,
- CHEMICAL ABSTRACTS, vol. 82, no. 5, 3 février 1975 Columbus, Ohio, US; abstract no. 28987, XP002031052 & RAIKHLIN ET AL.: "Clarifying action of the extract of mucosa...frog skin" BYULL. EKSP. BIOL. MED., vol. 78, no. 8, 1974, pages 114-117,
- CHEMICAL ABSTRACTS, vol. 85, no. 1, 5 juillet 1976 Columbus, Ohio, US; abstract no. 590, XP002031053 & RABADAN ET AL.: "effect of melatonin and two synthetic derivatives on amphibian skin color" ARCH. FARMACOL. TOXICOL., vol. 1, no. 3, 1975, pages 253-8,
- Pschyrembel Klinisches Wörterbuch, 257, neu bearbeitete Auflage (1994), Seite310: Depigmentierung
- Pschyrembel Klinisches Wörterbuch, 257, neu bearbeitete Auflage (1994), Seite 680: Hyperpigmentierung
- M.D. Rollag & M.R. Adelmann, 1992, Animal Reproduction Science, 30:67-89
- D. Sugden & S.J. Rowe, 1992, J. Cell Biol., 119:1515-1521

## Description

L'invention se rapporte l'utilisation de dérivés de la mélatonine comme agents de dépigmentation dans ou pour la préparation d'une composition cosmétique ou dermatologique par application topique sur la peau du visage et/ou du corps, dans le but de blanchir la peau ou de traiter les taches pigmentaires.

A différentes périodes de leur vie, certaines personnes voient apparaître sur la peau et plus spécialement sur les mains, des taches plus foncées et/ou plus colorées, conférant à la peau une hétérogénéité. Ces taches sont dues à une concentration importante de mélanine dans les kératinocytes situés à la surface de la peau. En effet, les mélanocytes situés dans la partie profonde de l'épiderme produisent de la mélanine et délivrent cette mélanine aux kératinocytes environnants, qui vont ensuite remonter à la surface de l'épiderme, chargés de mélanine.

Le mécanisme de formation de la pigmentation de la peau, c'est-à-dire de la formation de la mélanine est particulièrement complexe et fait intervenir schématiquement les principales étapes suivantes :

Tyrosine → Dopa → Dopaquinone → Dopachrome → Mélanine

La tyrosinase est l'enzyme essentielle intervenant dans cette suite de réactions. Elle catalyse notamment la réaction de transformation de la tyrosine en Dopa (dihydroxyphénylalanine) et la réaction de transformation de la Dopa en dopaquinone. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut ainsi être bloquée et assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de la toxicité qu'ils entraînent, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi l'hydroquinone, dont l'emploi est d'ailleurs limité légalement à une concentration de 2 %, est un composé particulièrement irritant et cytotoxique pour le mélanocyte, dont le remplacement, total ou partiel a été envisagé par de nombreux auteurs.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo. Pour ces dernières hyperpigmentations, à défaut de pouvoir repigmenter la peau lésée, on achève de dépigmenter les zones de peau normale résiduelle pour donner à l'ensemble de la peau une teinte blanche homogène.

L'utilisation de dérivés d'acide 2,5-dihydroxyphényl carboxylique ou de dérivés du benzofurane est décrite dans les demandes de brevet EP 0 524 108 et EP 0 526 302.

La demanderesse a maintenant trouvé que certains dérivés de la mélatonine agissent sur la pigmentation et les taches de la peau.

La mélatonine, ou N-acétyl-5-méthoxytryptamine est surtout connue pour son activité circadienne régulant la production d'hormones, en particulier pour son influence sur le rythme du sommeil. Elle a également été décrite pour son activité antioxydante et son utillisation en dermo-cosmétique pour améliorer l'apparence de la peau (JP 61 221 104; US 4 746 674), ou pour protéger la peau contre l'effet d'une irradiation aux rayonnements U.V. (EP 0 438 856; E. Bangha & al., Dermatology 191, [2], 176,1995). Différentes compositions topiques comprenant de la mélatonine pour un usage thérapeutique ou cosmétique ont été décrites, comme des lotions de toilettes, des crèmes ou des laits de toilette (JP 61 221 104).

Si la mélatonine peut moduler la production d'a-MSH au niveau central, et en conséquence la production de mélanine, la litérature enseigne que la mélatonine exogène est sans effet sur la pigmentation de la peau chez l'homme (D.B. McElhinney & al., J. Invest. Dermatol 102 (2), 1994, 258-9).

La présente invention a pour objet l'utilisation, comme agent de dépigmentation cutanée, dans une composition cosmétique ou pour la préparation d'une composition dermatologique, d'un dérivé de la mélatonine de formule (I): 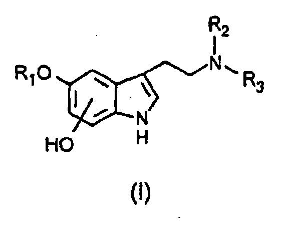 dans laquelle
R₁ représente un radical alkyle Inférieur,
R₂ représente un atome d'hydrogène ou un radical alkyle inférieur, et
R₃ représente un atome d'hydrogène ou un radical acyle inférieur,
le radical hydroxyle sur le noyau indole pouvant se situer en position 4, 6 ou 7, ses sels, solvates ou bioprécurseurs physiologiquement acceptables.

D'une manière avantageuse, il s'agit des dérivés de formule générale (Ia) 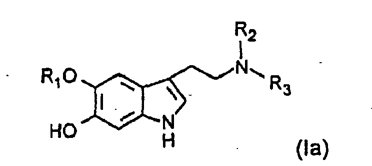 dans laquelle R₁, R₂ et R₃ sont définis ci-dessus et le radical hydroxyle est en position 6.

Il s'agit des dérivés de formule générale (I) et (Ia) dans lequelles R1 représente un radical parmi les radicaux alkyles en C1 à C4, linéaires ou ramifies, éventuellement substituées par un ou plusieurs halogènes (F, Cl ou Br), R2 représente un atome d'hydrogène ou un radical parmi les radicaux alkyles en C1 à C4, linéaires ou ramifies éventuellement substituées par un ou plusieurs halogènes (F, Cl ou Br) et R3 représente un atome d'hydrogène ou un radical acyle dont le reste alkyle est choisi parmi les radicaux alkyles en C1 à C4, linéaires ou ramifies, éventuellement substituées par un ou plusieurs halogènes (F, Cl ou Br).

D'une manière avantageuse, il s'agit des dérivés de formule générale (I) et (la) dans lesquelles R₁ représente un radical méthyle, R₂ représente un atome d'hydrogène, et R₃ représente un radical acétyle.

Par alkyle inférieur, on entend de préférence les radicaux méthyle, éthyle, propyle ou butyle. Cette définition s'applique au restes alkyles des radicaux acyles.

Par sel on entend tout sel d'addition d'un acide minéral ou organique physiologiquement acceptable, usuel pour des composés actifs en cosmétique ou en dermatologie, tels que des sels d'addition avec l'acide chlorhydrique, sulfurique, acétique, citrique, fumarique, hémisuccinique, maléique, etc.

Par bioprécurseur physiologiquement acceptable, on entend les dérivés aptes à libérer les composés de formule générale (I) ci-dessus une fois administrés, en particulier les esters, tels que les alkyl phosphates, alkyl sulfates ou acyles (par exemple acétate), ou les osides (en particulier glucosyl, mannosyl, fructosyl, N-acétylglucamine, galactosyl) du radical hydroxyle.

Les dérivés de la mélatonine sont utilisés, selon la présente invention, de préférence en quantité allant de 0,0001 à 10 % en poids et encore plus préférentiellement de 0,001 à 1 %.

L'application d'une composition contenant un dérivé de la mélatonine selon l'invention permet d'obtenir une nette diminution voire une disparition complète de la formation de taches de pigmentation. Sans préjuger du mode d'action des dérivés de la mélatonine, il semble que la dépigmentation est obtenue par l'application d'une quantité appropriée de dérivé de la mélatonine suffisante pour inhiber la tyrosinase des mélanocytes de la peau.

Aussi, l'invention a pour objet un procédé de traitement cosmétique des tachés de pigmentation de la peau consistant à appliquer sur la peau une composition contenant, comme agent de dépigmentation cutanée, un dérivé de la mélatonine de formule (I) telle que définie ci-dessus.

L'invention a encore pour objet l'utilisation d'un dérivé de la mélatonine de formule (I) telle que définie précédemment, pour la préparation d'un médicament destiné à traiter les taches pigmentaires, particulièrement en quantités suffisantes comme inhibiteur de la tyrosinase des mélanocytes de la peau.

Les compositions contenant les dérivés de la mélatonine utilisées selon l'invention peuvent présenter toutes les formes galéniques normalement utilisées pour une application topique, par exemple sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

De façon connue, les compositions cosmétiques ou dermatologiques utilisées de l'invention peuvent contenir des adjuvants habituels dans le domaine cosmétique ou dermatologique, tels que les émulsionnants, les gélifiants hydrophiles ou lipophiles, les actifs hydrophiles ou lipophiles, les conservateurs, les antioxydants, les parfums, les charges, les filtres et les matières colorantes. Les quantités de ces différents adjuvants sont celles classiquement utilisées dans les domaines cosmétique et/ou dermatologique, et par exemple de 0,01 % à 20 % du poids total de la composition. Ces adjuvants, selon leur nature, peuvent être introduits dans la phase grasse, dans la phase aqueuse et/ou dans les vésicules lipidiques.

Comme émulsionnants, on peut utiliser des émulsionnants eau-dans huile (E/H) ou huile-dans-eau (H/E) selon l'émulsion finale souhaitée.

Comme émulsionnants, on peut citer par exemple le stéarate de PEG-20, le stéarate de PEG-100, le Polysorbate 60 (Tween 60 vendu par la société ICI, le stéarate de sorbitan (Span 60 vendu par la société ICI) et le PPG-3 myristyl éther.

Le taux d'émulsionnant peut aller de 0,1 % à 15 % en poids, et de préférence de 0,5 % à 5 % en poids, par rapport au poids total de la composition.

On peut ajouter à la composition utilisée selon l'invention des coémulsionnants, par exemple en une quantité allant de 0,05 % à 10 % en poids par rapport au poids total de la composition. Comme coémulsionnant, on peut citer le stéarate de glycérol.

Dans les dispersions de vésicules lipidiques, l'émulsionnant peut être constitué par des vésicules de lipides ioniques et/ou non ioniques.

Comme huiles utilisables dans l'invention, on peut citer les huiles minérales, les huiles végétales (huile de tournesol, huile d'amande d'abricot, huile de karité), les huiles de synthèse, les huiles siliconées (cyclométhicone) et les huiles fluorées (perfluoropolyéthers). On peut ajouter à ces huiles des alcools gras (alcool stéarylique), des acides gras (acide stéarique) et des cires.

Comme gélifiants hydrophiles, on peut citer les polymères carboxyvinyliques, les polyacrylates ou polyméthacrylates de glycéryle, les polyacrylamides, les gommes naturelles (xanthane) et les argiles, et, comme gélifiants lipophiles, on peut citer les argiles modifiées comme les bentones, les sels métalliques d'acides gras comme les stéarates d'aluminium et la silice hydrophobe.

Comme actifs hydrophiles, on peut utiliser par exemple les protéines ou les hydrolysats de protéine, les acides aminés, les polyols, en particulier la glycérine ou le sorbitol, l'urée, l'allantoïne, les sucres et leurs dérivés, l'acide glycyrrhétinique.

Comme actifs lipophiles, on peut citer le tocophérol (vitamine E) et ses dérivés, les acides gras essentiels, les céramides, les huiles essentielles.

On peut aussi utiliser dans ces compositions des filtres UV à propriété lipophile ou hydrophile, les oxydes de titane et de zinc.

Ces compositions constituent notamment des crèmes de protection, de traitement ou de soin pour le visage, pour les mains ou pour le corps, des laits corporels de protection ou de soin, des lotions, gels ou mousses pour le soin ou le traitement de la peau, des lotions de nettoyage ou de désinfection, des compositions pour le bain, des fonds de teint et des crèmes teintées. Dans ces derniers cas, la composition contient des pigments.

### Etude "in vitro":

L'activité des dérivés selon l'invention comme agents de dépigmentation a été mise en évidence dans le test in vitro d'inhibition de la tyrosinase.

Selon ce test, on suit par spectrométrie visible à 475 nm la quantité de dopachrome formée au cours de la chaîne de réaction de transformation de la tyrosine en mélanines. Ces réactions sont catalysées in vitro par la tyrosinase de champignons, en présence d'un co-substrat réducteur (par exemple la L-dopa en petite quantité) pour initier la réaction d'hydroxylation de la L-tyrosine en L-dopa, laquelle est ensuite oxydée catalytiquement en dopaquinone puis en dopachrome.

On mesure donc la concentration en dopachrome formé au cours du temps en présence et en absence de l'inhibiteur. On exprime l'effet d'inhibition par l'abaissement de la quantité maximale de dopachrome formé (valeur de densité optique à 475 nm lue au plateau de la courbe) par rapport à la quantité obtenue en l'absence d'inhibiteur.

### Réactifs

A. Tampon phosphate 0,1 M pH 6,5 (Tween 20 à 1%)
B. Solution mère de L-tyrosine à 2.10⁻³ M dans A
C. Solution mère de L-dopa à 10⁻⁴ M dans A
D. Soluytion mère de tyrosinase de champignons à 2400 unités/ml dans A
E. Solution mère de l'inhibiteur à 10⁻² M dans A
Les solutions C et D sont à préparer le jour même.

### Résultats

| | |
|---|---|
| **Cuve de référence** | 3 ml de A |
| **Cuve d'essai** | 1 ml de B |
| | 0,1 ml de C |
| | 1,85 ml de A+E |
| | Homogénéiser et équilibrer à 25°C ajouter 0,05 ml de D |

Mélanger rapidement et observer la cinétique par la mesure de l'absorbance en fonction du temps.

Des essais ont été effectués avec la 6-hydroxymélatonine pour 0,1 et 0,2 ml de solution E. Dans les deux cas on observe une inhibition significative de l'activité de la tyrosinase par diminution du pourcentage de formation de dopachrome.

Des essais similaires effectué avec la mélatonine et des dérivés de la 5-hydroxytryptamine montrent une absence d'inhibition.

Les exemples suivants illustrent l'invention. Dans ces exemples, les proportions indiquées sont des pourcentages en poids.

### Exemple 1 : Emulsion H/E

| | |
|---|---|
| 6-hydroxy mélatonine | 0,05 % |
| Octyldodécanol | 5 % |
| Huile de tournesol | 11 % |
| EDTA | 0,05 % |
| Hydroxyde de sodium | 0,02 % |
| Gomme de xanthane | 0,2 % |
| Polyacrylamide/Isoparaffine/Laureth-7 (Sepigel 305 vendu par la société Seppic) | 0,9 % |
| Cyclométhicone | 5 % |
| Glycérine | 4 % |
| Polyacrylate de glycéryle à 2 % dans un mélange eau/glycérine (Lubrajel vendu par la société Guardian) | 5 % |
| Stéarate de glycérol | 0,6 % |
| Stéarate de PEG-100 | 0,6 % |
| Stéarate de PEG-20 | 1,2 % |
| Acide stéarique | 0,6 % |
| Alcool stéarylique | 1 % |
| Conservateurs | 0,3 % |
| Eau | qsp 100 % |

On obtient une crème fluide de couleur blanche à propriété dépigmentante.

### Exemple 2 : Emulsion H/E

| | |
|---|---|
| 6-hydroxy mélatonine | 0,1 % |
| Huile d'amande d'abricot | 10 % |
| Huile de karité | 7 % |
| PPG-3 myristyl éther | 5 % |
| Polysorbate 60 (Tween 60) | 2,5 % |
| Stéarate de Sorbitan (Span 60) | 2,5 % |
| Conservateurs | 0,2 % |
| Cyclométhicone | 4 % |
| Gomme de xanthane | 0,2 % |
| Polymère carboxyvinylique | 0,5 % |
| Triéthanolamine (neutralisant) | 0,5 % |
| Glycérine | 5 % |
| eau | qsp 100 % |

On obtient une bonne crème de jour dépigmentante.

## Revendications

1. Utilisation d'un dérivé de la mélatonine de formule (I): 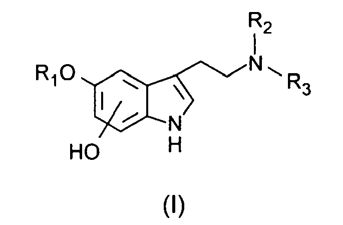 dans laquelle
R₁ représente un radical parmi les radicaux alkyles en C₁ à C₄, linéaires ou ramifiés, éventuellement substitués par un ou plusieurs halogènes (F, Cl, Br),
R₂ représente un atome d'hydrogène ou un radical parmi les radicaux alkyles en C₁ à C₄, linéaires ou ramifiés, éventuellement substituées par un ou plusieurs halogènes (F, Cl ou Br),
R₃ représente un atome d'hydrogène ou un radical acyle dont le reste alkyle est choisi parmi les radicaux alkyles en C₁ ou C₄, linéaires ou ramifiés, éventuellement substituées par un ou plusieurs ou plusieurs halogènes (F, Cl, Br),
le radical hydroxyle sur le noyau indole pouvant se situer en position 4, 6 ou 7, ses sels, solvates ou bioprécurseurs physiologiquement acceptables, comme agent de dépigmentation cutanée dans une composition cosmétique.

2. Utilisation selon la revendication 1, **caractérisée en ce que** le radical hydroxyle est en position 6.

3. Utilisation selon l'une des revendications 1 ou 2, **caractérisée en ce que** R₁ représente un radical méthyle, R₂ représente un atome d'hydrogène, et R₃ représente un radical acétyle.

4. Utilisation selon l'une des revendications 1 à 3, **caractérisée en ce que** le dérivé de mélatonine est utilisé en une quantité allant de 0,0001 à 10 % en poids par rapport au poids total de la composition.

5. Utilisation selon la revendication 4, **caractérisée en ce que** le dérivé de mélatonine est utilisé en une quantité allant de 0,001 à 1 % en poids par rapport au poids total de la composition.

6. Procédé de traitement cosmétique des taches de pigmentation de la peau consistant à appliquer sur la peau une composition contenant, comme agent de dépigmentation cutanée, un dérivé de la mélatonine de formule (I) telle que définie dans l'une des revendications 1 à 3.

7. Utilisation d'un dérivé de la mélatonine de formule (I) telle que définie dans l'une des revendications 1 à 3, pour la préparation d'un médicament destiné à traiter les taches pigmentaires.

8. Utilisation selon la revendication 7, **caractérisée en ce que** l'on utilise le dérivé de la mélatonine en quantités suffisantes pour inhiber la tyrosinase des mélanocytes de la peau.

## Claims

1. Use of a melatonin derivative of formula (I): 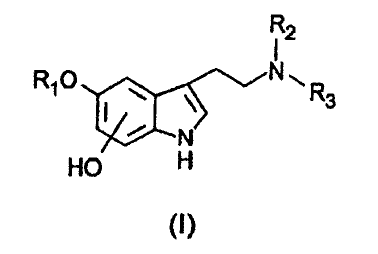 in which
R1 represents a radical chosen from linear or branched alkyl radicals C1 to C4, optionally substituted with one or more halogens (F, C1 or Br),
R2 represents a hydrogen atom or a radical chosen from linear or branched alkyl radicals C1 to C4, optionally substituted with one or more halogens (F, Cl or Br), and R3 represents a hydrogen atom or an acyl radical whose alkyl radical is chosen from linear or branched alkyl radicals C1 to C4, optionally substituted with one or more halogens (F, Cl or Br),
it being possible for the hydroxyl radical on the indole ring system to be in position 4, 6 or 7,
its physiologically acceptable salts, solvates or bioprecursors, as a skin depigmenting agent in a cosmetic composition.

2. Use according to Claim 1, **characterized in that** the hydroxyl radical is in position 6.

3. Use according to either of Claims 1 and 2, **characterized in that** R₁ represents a methyl radical, R₂ represents a hydrogen atom and R₃ represents an acetyl radical.

4. Use according to one of Claims 1 to 3, **characterized in that** the melatonin derivative is used in an amount ranging from 0.0001 to 10 % by weight relative to the total weight of the composition.

5. Use according to Claim 4, **characterized in that** the melatonin derivative is used in an amount ranging from 0.001 to 1 % by weight relative to the total weight of the composition.

6. Process for the cosmetic treatment of skin pigmentation blemishes, which consists in applying to the skin a composition containing, as skin depigmenting agent, a melatonin derivative of formula (I) as defined in one of Claims 1 to 3.

7. Use of a melatonin derivative of formula (I) as defined in one of Claims 1 to 3, for the preparation of a medicinal product intended for the treatment of pigmentation blemishes.

8. Use according to Claim 7, **characterized in that** the melatonin derivative is used in amounts which are sufficient to inhibit the tyrosinase of skin melanocytes.

## Patentansprüche

1. Verwendung eines Melatoninderivats der Formel (I): 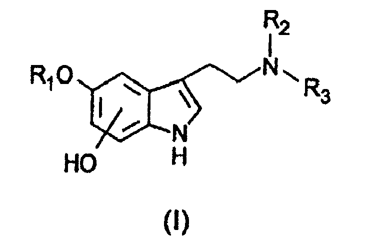 worin bedeuten:
R₁ eine Gruppe, die unter den geradkettigen oder verzweigten C₁₋₄₋Alkylgruppen ausgewählt ist, die gegebenenfalls mit einem oder mehreren Halogenatomen (F, Cl oder Br) substituiert sind,
R₂ ein Wasserstoffatom oder eine Gruppe, die unter den geradkettigen oder verzweigten C₁₋₄-Alkylgruppen ausgewählt ist, die gegebenenfalls mit einem oder mehreren Halogenatomen (F, Cl oder Br) substituiert sind, und
R₃ ein Wasserstoffatom oder eine Acylgruppe, deren Alkylgruppe unter den geradkettigen oder verzweigten C₁₋₄-Alkylgruppen ausgewählt ist, die gegebenenfalls mit einem oder mehreren Halogenatomen (F, Cl oder Br) substituiert sind,
wobei sich die Hydroxygruppe an dem Indolring in 4-, 6- oder 7-Stellung befindet,
und ihrer Salze, Solvate oder Bioprecursoren, die physiologisch akzeptabel sind,
als Wirkstoffe zur Depigmentierung der Haut in einer kosmetischen Zusammensetzung.

2. Verwendung nach Anspruch 1, **dadurch gekennzeichnet, daß** sich die Hydroxygruppe in 6-Stellung befindet.

3. Verwendung nach einem der Ansprüche 1 oder 2, **dadurch gekennzeichnet, daß** die Gruppe R₁ Methyl bedeutet, die Gruppe R₂ ein Wasserstoffatom ist und die Gruppe R₃ Acetyl bedeutet.

4. Verwendung nach einem der Ansprüche 1 bis 3, **dadurch gekennzeichnet, daß** das Melatoninderivat in einem Mengenanteil von 0,0001 bis 10 Gew.-%, bezogen auf das Gesamtgewicht der Zusammensetzung, verwendet wird.

5. Verwendung nach Anspruch 4, **dadurch gekennzeichnet, daß** das Melatoninderivat in einem Mengenanteil von 0,001 bis 1 Gew.-%, bezogen auf das Gesamtgewicht der Zusammensetzung, verwendet wird.

6. Verfahren zur kosmetischen Behandlung von Pigmentflecken der Haut, das darin besteht, auf die Haut eine Zusammensetzung aufzutragen, die als Wirkstoff zur Depigmentierung der Haut ein Melatoninderivat der Formel (I) nach einem der Ansprüche 1 bis 3 enthält.

7. Verwendung eines Melatoninderivats der Formel (I) nach einem der Ansprüche 1 bis 3 zur Herstellung eines Arzneimittels, das zur Behandlung von Pigmentflecken vorgesehen ist.

8. Verwendung nach Anspruch 7, **dadurch gekennzeichnet, daß** das Melatoninderivat in Mengen verwendet wird, die ausreichend sind, um die Tyrosinase der Melanocyten der Haut zu inhibieren.
